# EUROPEAN PATENT APPLICATION

(11) **EP 3 671 759 A1**
(43) Date of publication of application: **24.06.2020**
(21) Application number: 18214568.0
(22) Date of filing: 20.12.2018
(51) Int. Cl.: G16H 50/20, A61B 5/00, A61B 5/11, G08B 21/04

(54) **METHOD FOR MONITORING A USER, METHOD FOR STORING AND/PROVIDING PERSONAL DATA OF A USER AND MONITORING SYSTEM**

(71) Applicant: Nively, 06000 Nice (FR)
(72) Inventor: Conti, Giuseppe, 06000 Nice (FR); Evdokimos, Konstantinidis, 06000 Nice (FR)
(74) Representative: Tergau & Walkenhorst Patentanwälte PartGmbB

(57) **Abstract**

Method for monitoring a user, whereby a plurality of conditions of a user is defined, and whereby each condition is related to a level of consent, comprising the steps
• continuously monitoring said user;
• during said continuous monitoring, detecting physiological and/or cognitive states and/or critical events;
• building a model for the condition of said user from said detected states and events;
• assessing if a change in the physiological and/or cognitive condition and/or a critical event, especially within a given period of reference, has occurred and/or is about to occur, and, if a change and/or critical event has occurred, prompting said user for re-consent by the user or by its legally appointed proxies, whenever the user is unable to consent, and/or prompting for explicit adjustment of previous consent due to an adjustment of the required degree of monitoring.

## Description

### Field of the invention

The invention concerns a method for monitoring a user. It also concerns a method for storing and/or providing personal data of a user and a monitoring system ensuring this is done lawfully through explicit provision of informed consent over time.

### Background Art

According to Eurostat (June 2016) on 1st January 2015, 18,9% of the EU-28 population (508,5 million) was aged 65+ with an increase of 0,4% compared with the previous year and an increase of 2.3% compared with 10 previous years. Such a rising older population is subject to varying levels of cognitive and functional impairments that require assistance from caregivers, be these formal or informal. Disturbances of executive functions are among the most common aspects of cognitive impairment and involve capacity to concentrate, communicate/use language, remember, plan and/or direct activity, monitor current activity, to name but the most relevant aspects. Cognitive impairment is often accompanied by psychomotor processing, persistent impairments of executive functions, which are associated with loss of social autonomy and inability to return to work or to take care of themselves independently. Many older adults with cognitive and physical impairments require constant care, either at a formal institution or in the form of homecare assistance. However, all analysts, as well as governments, agree upon the evidence that our society will not be able to afford the current care model with the available funding.

The problem is that older adults typically prefer living at their homes as long as possible. However, they often need to be institutionalized due to the problems associated with cognitive impairment, neurodegenerative disorders, functional disability, and other frailty indicators that may present along with deficits in memory or executive function (i.e. working memory, reasoning, planning, problem-solving). The result of such combination of factors may lead to increasing number of falls, failure to take necessary medication or disorientation (in time and space).

The challenge that needs to be addressed is that homecare as well as institutional care, can benefit today from a range of existing technologies including smartphones, sensors, etc., however, their effectiveness is limited by the sense of fear and uncomfortableness that many older adults feel. Most current technologies, in fact, are often not designed for older adults, least of all for users with memory impairments. Most systems are not offering real care neither they are empowering personalized interventions. Furthermore, many are unable to seam-lessly "blend" within the person's daily life and living environment. Lastly, they are rarely engineered to preserve or improve physical and cognitive performances or to preserve mental health and social well-being of older adults. This limitation becomes a major barrier, severely limiting use of technological assistance in a home environment.

The US 9,519,969 B1 discloses a method and a system for monitoring an individual. A plurality of 3D motion and sound sensors are located in the dwelling and provide data to a computerized monitoring system. The sensors are configured to recognize one or more biometric identifiers of the individual being monitored. When the monitoring system detects that the individual has fallen or gestured, a computerized communication system contacts the individual to determine the need to send assistance to help the individual.

In order to detect critical situations of the user, not necessarily senior, the user should be monitored by capturing video and audio data which upon analyzation yields reliable information on the status of the user. The monitoring of the user should, however, be based on current standards of privacy.

The object of the invention is therefore to provide a method for monitoring a user which assures a monitoring method providing user safety and at the same time respecting the user consent at all times. Furthermore, an improved monitoring system is provided.

In a first aspect, the invention relates to a method for monitoring a user, whereby a plurality of conditions of a user is defined, and whereby each condition is related to a level of consent, comprising the steps:
- continuously monitoring the user;
- during this continuous monitoring, detecting physiological and/or cognitive states and/or critical events;
- building a model for the condition of the user from said detected states and events;
- assessing if a change in the physiological and/or cognitive condition and/or a critical event has occurred, or is about to occur, especially within a given period of reference has occurred, and, if a change and/or critical event has occurred, prompting the user for re-consent by the user or by its legally appointed proxies, whenever the user is unable to consent, and/or prompting for explicit adjustment of previous consent due to an adjustment of the required degree of monitoring.

Preferred embodiments of the invention are disclosed in the dependent claims and in the description.

The invention is based on the consideration that monitoring of a user should in no situation be performed without the consent of the user. However, change in detected physiological, cognitive, psychological conditions (e.g. a senior alone, a solo worker, a person affected by various mental health conditions or cognitive syndromes, etc.) may require change of monitor patterns or metrics, which, in turn, would require re-consent. Frequently asking for consent is unpractical and my cause frustration of the user or it could be unpractical (e.g. a worker operating alone). This could result in a withdrawal of the user from using the system. Furthermore, degrading conditions of user, especially seniors or patient affected by cognitive, behavioural or mental health conditions, however, due to possibly fast evolving physical and cognitive conditions, may change perception of the situation that would change willingness to consent by the user. For instance, typically the acceptance of a more intrusive monitoring system becomes significantly higher after a dramatic episode, most typically after a fall in case of seniors or other events or crisis in case of behavioural, cognitive or mental health conditions. In this case the subject or her/his proxy may become more inclined to relaxing privacy protection in favour of higher control for safety reasons.

Moreover, the physical and cognitive conditions of the user may decade significantly over a relatively short time due to fast evolving clinical conditions or clinical episode, for instance a stroke, that may leave the user unable to consent.

It is therefore highly desirable to provide as much safety as possibly while at the same time keep the intrusion necessary at a low level.

Applicant has found that these demands can be met by making the request or reformulation of consent dependent on a change of the condition of the user. As long as the condition of the user remains essentially the same, no consent is requested. Only if the condition / state of the user changes in a way that an increased level of monitoring leads to a valuable increase of safety, the user is prompted for a re-consent or reformulation of consent, the latter in case additional monitoring parameters would be required to be recorded and analysed, based on the new detected state.

The system is therefore figuratively speaking minimally intrusive and only interferes or interacts with the user when it is necessary for obtaining a considerably increase in the safety of the user. The user is preferably prompted to provide explicit consent through computerised interfaces other user interfaces (smart television, mobile devices, augmented or projected reality interfaces, holographic interfaces, etc.) while consent is collected in an ambiguous say by using multi-modal interactions with explicit multiple confirmation (for instance through an explicit gesture, preferably plus voice confirmation).

Explicit consent by a proxy is preferably requested through several means, including push message to their App, delivery of SMS or through automatic phone call to pre-configured list of numbers.

Critical events about to occur may be emerging either through detection of significant degradation of physical performance, abnormal posture (e.g. the person crouching and holding on a chair for help).
The prompting for reconsent is preferably conducted if conditions resulting from monitoring may indicate the user not being any more aware of having previously consented.

If an explicit condition of danger is manifested by the user, for instance him / her calling for help explicitly with his / her voice, and after not having received any counter order following a message by the system informing that an emergency has been detected, the system preferably regards this event as explicit consent and therefore proceeds to extraction all key information which may be required (including capturing of images, if applicable) to generate and send alert to pre-authorized persons.

Preferably said consent is related, especially only related to, data management and/or data safety.

Advantageously, monitoring is performed by movement or behavioural detection, especially from the group of: 3-D depth sensor, video camera, audio recording units with microphones, wearable devices or other wireless motion and/or activity and/or intention detection technologies.

Changes in the condition preferably comprise at least a change in a physiological and/or cognitive and/or behavioural pattern.

Preferably, a pattern comprises at least one of walking speed, gait, walking patterns, habit patterns, gesture ranges, biological cycles, emotion detection for a prescribed time interval and/or interaction/reaction time.

Randomization is crucial for encryption schemes to achieve semantic security, a property whereby repeated usage of the scheme under the same key does not allow an attacker to infer relationships between segments of the encrypted message. For instance, for block ciphers, the use of an IV (initialization vector) is described by the modes of operation. Randomization is also required for other primitives, such as universal hash functions and message authentication codes based thereon. (source: Wikipedia)". True randomisation is not easy achieved, and it is usually achieved through complex mathematical formulas which based on use of functions with high entropy.

Applicant has found that a very advantageous randomization can be achieved by collecting personal data based on analysis of user's movements, by extracting "skeletal" geometry and analysing their configuration to assess conditions of danger. Skeletons are defined through position in space (X, Y, Z) of body joints. In a preferred case, they include multiple positions (usually between 20-30) such as head, right shoulder, left shoulder, right elbow, etc. A high-entropy true randomised value can be created deriving it from skeleton configuration at any given time. For example, in case of a fall, a mathematical operator can be applied to derive a randomised value that can be used to store information related to the sequence of captured skeletal movements which may be used later by the doctor to understand the dynamic of the fall. The skeleton configuration changes continuously therefore it is naturally a value with high entropy, as collecting data at centimetre or sub-centimetre precision at high frequency (typically above and beyond 30 Hz) ensures that the data will be always different.

In a preferred embodiment, personal data of the user is stored and/or provided, whereby the user is captured by a 3D capturing and/or video device and/or other wireless or wearable technology, and whereby body position and/or skeletal configuration data is obtained from the capture data, whereby from the body position and/or skeletal configuration data at least one quantity is deduced which is employed for storing the personal data.

Preferably, a high-entropy randomized value is derived from a mathematical function which uses, as coefficient, parameters calculated starting from the body position and/or skeletal configuration data which is used to store and/or provide the personal data.

Advantageously, for storing and/or providing said personal data a cryptographic algorithm is used, and whereby from the body position and/or skeletal configuration data a crypto value is obtained (e.g. a signature vector comprising of multiple measurements in space, time, frequency, etc. characterised by high level of entropy) which serves as an input value for the cryptographic algorithm, and whereby the algorithm depends on this value, or, in case of multiple users, the crypto value is derived by "group encryption" techniques.

The body position and/or skeletal configuration data is used to create a key pair in a public (asymmetric) key infrastructure.

Preferably, visual and/or audio data and/or other body position data, including direct recording of skeletal structure as well as analysed information regarding gait, libs movement ranges, indicators derived from analysis of structured actions (e.g. Tinetti test or Performance-Oriented Mobility Assessment - POMA tests), which can be retrieved on request, whereby parts of the data of a person are removed and/or replaced by other data dependent on the consent of this person.

Advantageously, corresponding visual data is replaced by 3D depth data or otherwise, for instance blurred or masked visual data to prevent people who have not given consent to become recognisable.

In a further aspect, the invention relates to a monitoring system for monitoring at least one user, comprising at least one video, 3D or other wireless or wearable sensing system for obtaining movement depth data of one o more users in real time, and comprising an analyzing unit for analyzing these data in real data, further comprising a crypto unit for encrypting said data, whereby these units are configured for conducting a method described above. The units described above can be realized in hardware and/or software. They can be realized as modules in a common control unit which is configured to conduct a method described above.

Advantageously, the monitoring system comprises an alarm unit, whereby if the control unit detects a critical or potentially critical condition, and/or conditions which may lead to emergence of such critical condition in the near future, the alarm unit issues an alarm.

Preferably, the monitoring system comprises a temporary storing unit being configured to record images (RGB or infrared or depth data or in other forms) and/or other body movement or skeletal configuration information, whereby captured data is stored for a predefined time interval in the temporary storing unit, and whereby older data is deleted from the temporary storing unit.

Preferably the predefined time interval lies between 10 seconds and 30 seconds, especially preferably the time interval is 15 seconds.

The monitoring system preferably comprises a main storage unit, whereby if at least one condition is fulfilled, data (which has been captured) is transferred from the temporary storage unit to the main storage unit. Examples include a fall by a person in a nursing home who has cognitive and memory impairment which manages to rise up without informing anyone of the event, who later is discovered to have injury (caused by the fall). In this case having the possibility to understand the event occurred to the person is key to define the most suitable therapy.

Preferably visual and/or audio data and/or other body position or skeletal configuration data can be retrieved on request, whereby parts of the data of a person are removed, processed and/or replaced by other data dependent on the consent of this person.

Advantageously, corresponding visual data is replaced by 3D depth data or otherwise masked visual data of the user(s) to prevent people who have not given consent to become recognisable.

The main storage unit preferably is accessible by user credentials and/or other strong authentication means.

The advantages of the invention are especially as follows. By interacting with the user for an adjustment or reconfirmation of consent only in situations where the condition of the user has changed, the user is provided with a high safety while at the same time her or his quality of life is not reduced by an intrusive system nor such re-consent impedes his or her current task (e.g. solo worker carrying on a compelling or dangerous task). Due to this criterion, when an interaction with the user regarding consent is conducted, the acceptance of the system by the user can be increased since the user learns that the system prompts her or him or their proxies for consent only in situations of need and does not interfere during normal routine.

Direct consent can be prompted in various forms, including but not limited to projected augmented reality or holographic interfaces, while direct consent is preferably collected through multi-modal interaction. The method can be employed in a variety of applications such as monitoring seniors, patients or other groups of persons, for example prisoners, solo workers, persons affected by mental disorders, persons affected by autism spectrum disorder, etc.

By employing skeleton information of the user for data encryption, an optimized randomization seed or key is provided which is truly random, highly entropic, and additionally user-specific.

### Brief description of the drawings

Further characteristics and advantages of the present invention will be highlighted in greater detail in the following detailed description of preferred embodiments of the invention, provided with reference to the enclosed drawings and given as an indication and not for limiting purposes.

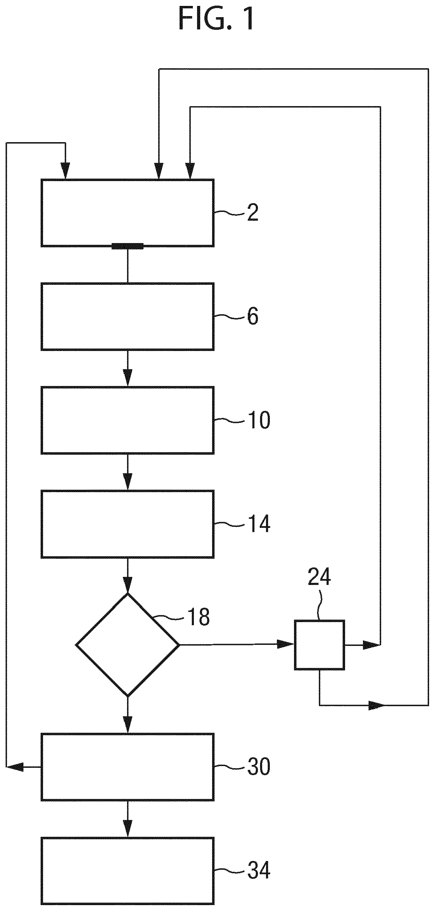

In particular, the attached drawings are included to provide a further understanding of the invention and are incorporated in and constitute a part of this specification. The drawings together with the description explain the principles of the invention. In the drawings, corresponding characteristics and/or components are identified by the same reference numbers. In these drawings, schematically
FIG. 1 shows a flow chart of a method for monitoring a user in a first preferred embodiment;
FIG.2 shows a diagram of a method for monitoring a user in a second preferred embodiment; and
FIG. 3 shows a monitoring system in a preferred embodiment.

In a box 2 of a flow chart of a preferred embodiment of monitoring a user, the user is continuously monitored. This monitoring is performed by employing, for instance, a video camera and a 3d-depth-field sensor. The monitoring of box 2 is conducted continuously. The pictures and 3d depth fields captured by these devices are analyzed in a box 6 for detection of physiological and cognitive states and critical events.

Physiological states which are monitored are especially depression, mood disorders, etc. Cognitive states which are monitored are especially degradation of coordination and response. Critical events which are monitored are especially falling of the person, night wandering, significant variation in gait patterns, including horizontal accelerations, walking speed, feet rising level, etc.

From these detected states and events, in a box 10, a model for the condition of the user is built where a set of indicators are used to define a vector state or to perform pattern analysis based on Al techniques, indicating emergency of dangerous conditions.

In a box 14, it is assessed if a change in the physiological and/or cognitive condition has occurred by assessing abnormal variation of typical values in typical conditions or emergency of new patterns, e.g. change in rhythm when carrying on ADL - Activities of Daily Living, the user spending prolonged time at the window, etc.

In a decision 18, it is decided if the change in the condition requires a change in the degree of monitoring of the user. If this is the case, the method branches to a box 24, in which the user is prompted for explicit adjustment of consent. In this way, the user explicitly has to agree with the increased and/or enhanced degree of monitoring.
The system requests the user to record additional or reduced set of data, explaining the reason to do so (the change in condition and which may relate to increased/lowered danger). Requests for consent are formulated by a graphical user interface, projected or augmented reality interface, holographic interface supported by voice. Provision of content is given through multi-modal interaction, including gestures, voice, etc. with the system explicitely asking for multiple confirmation.

If the user has expressed her/his consent, the method branches to box 2 in which the user is monitored with the increased and/or enhanced monitoring level. For instance, a lowering average walking speed, which may indicate a degradation of physical performance, may prompt a request for analyzing gait for more accurate analysis.

If the user does not provide his consent, the method branches to box 2 and continues to monitor the user with the monitoring level as before. In case of severe degradation of conditions, with consequent very high risk of danger, additional requests are prompted to the user while alert messages are delivered (if consent has been given) whenever a true emergency is detected. If an explicit condition of danger is manifested by the user, for instance him / her calling for help explicitly with his / her voice, and after not having received any counter order following a message by the system informing that an emergency has been detected, the system preferably regards this event as explicit consent and therefore proceeds to extraction all key information which may required (including capturing of images, if applicable) to generate and send alert to pre-authorised persons.

If in decision 18 it is recognized that the user not being any more aware of having previously consented, the method branches to a box 30 in which the user is prompted for reconsent. A request could be prompted on a screen or it could be asked by a virtual agent being rendered through augmented, projected or holographic visualisation means. The user could provide consent, as requested by the system, for instance by rising their right arm twice, followed by explicit voice consent (other forms of mixed multi-modal consent would apply).

If the user reconsents, the method continues inbox 2. If the user does not reconsent, the method branches to a box 34 in which the user keep monitoring at the current level, making additional choices regarding further request for reconsent (whenever conditions would increasingly require so),

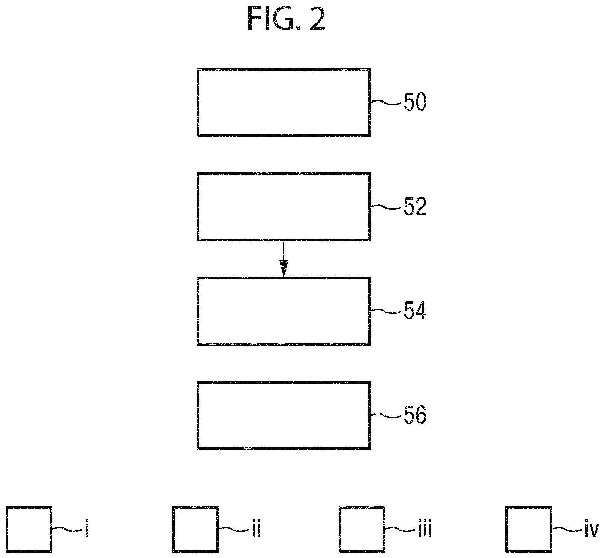

In the following, an example in a nursing home is described in relation to FIG. 2. The following pre-conditions are given: A monitoring system is installed in each bedroom of a nursing home. It is capable to detect, especially through a 3D sensor, movement of persons and conditions of risks by analysing 3D depth data in real time. A senior (aware to consent and therefore with no proxy) which in this case corresponds to the user has consent to collection and analysis of his personal data (skeleton and depth images from a sensor in the room including "clear" (RGB) and depth images) in case of fall.

At least two nurses Alice and Bob in the nursing home are responsible for caretaking of the senior.

A first nurse Alice has not consented to record and processing of her RGB images but only depth images. Instead, consent to collection of information related to her job tasks is permanently granted by signing of a collective contract between the union and the management of the nursing home, for instance: the system can record (without asking for consent) the when the nurse confirms through an App (on smartphone) that she has taken in charge a given alert at a given time, as well as the fact she has arrived to the room where the problem was detected, is recorded by her swiping her smartphone on a NFC tag.

A second nurse, Bob, has instead consented to recording of images (both depth images and visible images) but not to recording to sound.

The monitoring system (box 50) continuously monitors the senior and stores the last 15 seconds images (both depth images and RGB images) in memory and starts recording them on a file ready to be sent to the central system once an event occurs. This way it becomes possible to understand the event dynamics later. Recording continues for 3 minutes after the event to be able to assess how reaction to the event has been provided.

The monitoring system from the captured video and or depth data extracts, respectively, for each person skeleton data.

The following scenario can occur. The senior falls while his room, which is a critical event being detected by the monitoring system in box 52. An alert is generated (box 54) and sent to the smartphones of the various nurses in the nursing home, also to nurses Alice and Bob.

Alice picks the alert and confirms she has taken it into account, deciding to go to the room in which the senior resides to intervene. Once she arrives, which is detected by the monitoring system, she swipes her smartphone to the NFC tag to confirm she has arrived in this room and this information is logged (alternatively an automatic indoor location system could record her position in real time and store the information).

Once arrived, understanding the seriousness of the situation, nurse Alice presses a button on her smartphone asking for additional help or asks for help as speech recognition systems detects her intention and sends request for help. Nurse Bob picks the new requests and arrives 60 seconds later in the room. The monitoring system detects (box 56) that a new person (Bob) has entered the room (for instance the use of NFC by the nurse, is used to understand that that specific nurse has entered the room or through an indoor location-based system).

Recording of images and skeletons is ongoing by the monitoring system and runs as follows:
i. RGB images are never recorded by the monitoring system since neither of the 3 persons (senior, Alice, Bob) have consent to this.
ii. Depth images are recorded by the monitoring system as follows:
   1. Based on tracking of skeleton data, the portion of images from nurse Alice are removed whenever it is not overlapping to Nurse Bob or the senior. In the latter case (removing the image would remove important information from the other users) it is encrypted with a key which uses skeleton data of Nurse Alice at that time.
   2. Portion of images from nurse Bob are encrypted with a specific key which uses skeleton data of nurse Bob at that time (to increase security).
   3. Portion of images from the senior are encrypted with a specific key which uses skeleton data of the senior.
   4. For each image the rest of the portion of the image (outside the areas of the user) is recorded with Group Encryption (GE). (See for example: "Group encryption (GE) is the encryption analogue of group signatures. It allows a sender to verifiably encrypt a message for some certified but anonymous member of a group. The sender is further able to convince a verifier that the ciphertext is a well-formed encryption under some group member's public key"; source: http://perso.enslyon.fr/benoit.libert/traceable-enc-PKC-full-version.pdf)
   5. Any common environmental data (e.g. sound, etc.) are recorded by using GE.
iii. Sometime later: The doctor of the nursing home wants to analyse the dynamic of the fall, for instance to understand it is always related to a specific condition (e.g. weak right end, tripping, etc.). He accesses the monitoring system providing his/her credentials.
iv. The monitoring system automatically accesses the consent state (this may change dynamically as previous item) and returns back a de-crypted video stream where:
   1. Portions of the images where Alice was captured have been removed and replaced with the corresponding section taken from depth images (as this is allowed by the current consent state of Alice) on top of which skeletons are rendered to show the dynamics (as this is allowed by current consent state).
   2. Sound is also available.
   3. The doctor continues playing the video.
   4. Once Bob arrives, audio goes mute as she has not given consent to recording of audio and therefore was stopped by the system accordingly. Images of Bob are instead visible and the skeleton is rendered in overlay (as this is allowed by current consent state).

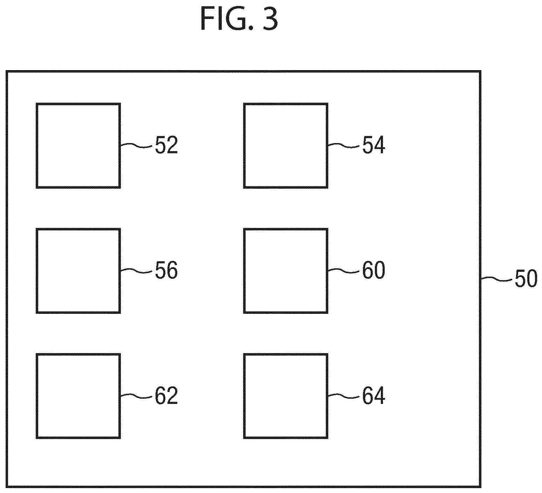

In FIG. 3, a monitoring system 50 is shown in a preferred embodiment. The monitoring system 50 comprises a sensing system 52 which comprises a video camera and a depth sensor for obtaining depth information on a user. The sensing system 52 also comprises an audio recording device for capturing audio data. The audio recording device can comprise one or more microphones. All captured data is preferably stored in persistent memory of a temporary storing unit 60 such as hard drives or SSD.

The monitoring system 50 further comprises an analyzing unit 54 in which the data captured by the sensing system 52 is - preferably after preprocessing - analyzed. In the analyzing unit 54, preferably a skeleton is extracted from the captured data in real time.

The monitoring system 50 further comprises a crypto or cryptography unit 56 in which the captured data is encrypted. The crypto unit 56 in a preferred variant uses skeleton data which has been generated in the analyzing unit 54 for encrypting visual and/or audio data.

Data is stored in the temporary unit only for a prescribed time or predefined time interval. In this way, the probability of data leaking out of the system is reduced since data are only present for a certain time interval.

The monitoring system 50 besides the temporary storing unit 60 also comprises a main storing unit 62. During regular operation, especially if no critical event or physiological and/or cognitive state change occurs, data is only stored in the temporary storing unit 60 temporarily.

If, however at least one condition is fulfilled, data is transferred from the temporary storing unit 60 to the main storing unit 62. Such a condition is especially a change of physiological and/or cognitive state and/or a critical event such as a fall or an unconscious or (potentially) hazardous condition. Only if such a condition is fulfilled, data which encompasses this situation is transferred to the main storing unit 62. This way the relevant data can be analyzed at a later stage by a doctor in order to assess the possible background / reasons for the occurrence of the cond i-tion change / critical event.

The monitoring system 50 moreover comprises an alarm or alarm unit 64 which issues an alarm and/or alert. Such an alarm is possibly a radio signal and/or acoustic and/or visual signal. Preferably it is a signal which is sent to a main unit and/or staff/personnel which is capable of corresponding action in such a scenario.

Units 52, 54, 56, 60, 62, 64 are configured to conduct a method described above. To this end, the method is preferably implemented as software instructions in the respective units. These units can be incorporated as hardware/software modules in a common control unit.

## Claims

1. Method for monitoring a user, whereby a plurality of conditions of a user is defined, and whereby each condition is related to a level of consent, comprising the steps
• continuously monitoring said user;
• during said continuous monitoring, detecting physiological and/or cognitive states and/or critical events;
• building a model for the condition of said user from said detected states and events;
• assessing if a change in the physiological and/or cognitive condition and/or a critical event, especially within a given period of reference, has occurred and/or is about to occur, and, if a change and/or critical event has occurred, prompting said user for re-consent by the user or by its legally appointed proxies, whenever the user is unable to consent, and/or prompting for explicit adjustment of previous consent due to an adjustment of the required degree of monitoring.

2. Method according to claim 1, whereby said consent is related, especially only related to, data management and/or data safety.

3. Method according to claim 1 or 2, whereby monitoring is performed by movement or behavioural detection, especially from the group of: 3-D depth sensor, video camera, audio recording units with microphones, wearable devices or other wireless motion and/or activity and/or intention detection technologies.

4. Method according to one of the claims 1 to 3, whereby changes in the condition comprise at least a change in a physiological and/or cognitive and/or behavioural pattern.

5. Method according to claim 4, whereby a pattern comprises at least one of walking speed, gait, walking patterns, habit patterns, gesture ranges, biological cycles, emotion detection for a prescribed time interval and/or interaction/reaction time.

6. Method according to one of the claims 1 to 5, whereby personal data of said user is stored and/or provided, whereby said user is captured by a 3D capturing and/or video device and/or other wireless or wearable technology, and whereby body position and/or skeletal configuration data is obtained from the capture data, whereby from said body position and/or skeletal configuration data at least one quantity is deduced which is employed for storing said personal data.

7. Method according to claim 6, whereby a high-entropy randomized value is derived from a mathematical function which uses, as coefficient, parameters calculated starting from said body position and/or skeletal configuration data which is used to store and/or provide said personal data.

8. Method according to claim 7, whereby for storing and/or providing said personal data a cryptographic algorithm is used, and whereby from said body position and/or skeletal configuration data a crypto value is obtained which serves as an input value for the cryptographic algorithm, and whereby said algorithm depends on this value, or, in case of multiple users, the crypto value is derived by "group encryption" techniques.

9. Method according to one of the claims 6 to 8, whereby said body position and/or skeletal configuration data is used to create a key pair in a public (asymmetric) key infrastructure.

10. Monitoring system (50) for monitoring at least one user, comprising at least one video, 3D or other wireless or wearable sensing system (52) for obtaining movement depth data of one or more users in real time, and comprising an analyzing unit (54) for analyzing these data in real time, further comprising a crypto unit (56) for encrypting said data, whereby said units are configured for conducting a method according to one of the previous claims.

11. Monitoring system according to claim 10, further comprising an alarm unit (64), whereby if said control unit detects a critical or potentially critical condition, said alarm unit (64) issues an alarm.

12. Monitoring system according to claim 10 or 11, comprising a temporary storing unit (60) being configured to record images and/or other body movement or skeletal configuration information, whereby captured data is stored for a predefined time interval in said temporary storing unit (60), and whereby older data is deleted from said temporary storing unit (60).

13. Monitoring system according to claim 12, comprising a main storage unit (62), whereby if at least one condition is fulfilled, data is transferred from said temporary storage unit (60) to said main storage unit.

14. Monitoring system according to one of the claims 1 to 13, whereby visual and/or audio data and/or other body position or skeletal configuration data can be retrieved on request, whereby parts of the data of a person are removed, processed and/or replaced by other data dependent on the consent of said person.

15. Monitoring system according to claim 14, whereby corresponding visual data is replaced by 3D depth data or otherwise masked visual data of the user(s) to prevent people who have not given consent to become recognisable.
